# EUROPEAN PATENT SPECIFICATION

(11) **EP 1 195 135 B1**
(45) Date of publication and mention of the grant of the patent: **11.05.2005**
(21) Application number: 01660187.4
(22) Date of filing: 05.10.2001
(51) Int. Cl.: A61B 5/024

(54) **Wrist-worn device**
Am Handgelenk tragbare Vorrichtung
Dispositif porté au poignet

(30) Priority: 06.10.2000 FI 20002209
(43) Date of publication of application: 10.04.2002
(73) Proprietor: Polar Electro Oy, 90440 Kempele (FI)
(72) Inventor: Nissilä, Seppo, 90550 Oulu (FI)
(74) Representative: Antila, Harri Jukka Tapani

(56) References cited:
- EP-A- 1 020 161
- WO-A-90/00366
- DE-A- 2 824 899
- FR-A- 2 595 480
- US-A- 5 840 039
- US-A- 5 876 346
- PATENT ABSTRACTS OF JAPAN vol. 017, no. 037 (C-1019), 25 January 1993 (1993-01-25) & JP 04 253839 A (MITSUBISHI ELECTRIC CORP), 9 September 1992 (1992-09-09)

## Description

### FIELD OF THE INVENTION

The field of application of the invention comprises wrist-worn devices, such as heart rate monitors, wrist-worn computers, or the like. The invention particularly relates to the structure of the wrist-worn device in question.

### BACKGROUND OF THE INVENTION

A heart rate monitor is a device for measuring the heart rate of an exerciser during a sports performance. Heart rate monitor solutions typically comprise an electrode belt placed on the chest to measure an electric signal produced by a heartbeat (see EP 1 020 161). The electrode belt transmits the measured heart rate information in a wireless manner to a wrist-worn device which processes the received signal to display the heart rate, and possibly other heart rate parameters, on the display of the wrist-worn device. Heart rate monitors are often used in extremely demanding conditions requiring resistance and reliability of the device as it may be subjected to hard wear, dampness and movement, for example. The wrist-worn device serves as a user interface for the user and is thus further required to provide ease and convenience of use and display characteristics ensuring good visibility.

Prior art wrist-worn devices included in heart rate monitors comprise a flexible wristband, the ends of which can be attached to and detached from one another and which allow the wristband to be tightened around the wrist to a desired tightness. The wristband is provided with a display unit comprising functions needed for using the heart rate monitor, such as input means and a display. In the prior art, the display unit is a rigid structure separate from the wristband. The display unit may be attached to the wristband in different known manners used in wrist-watches, such as pins provided in the display unit or different male-female type connections.

The prior art structures for wrist-worn devices are rigid and thereby unpleasant to use. To manufacture the display unit and the wrist-worn device as separate units causes added costs at the manufacturing phase. Further, the display of information to the user is limited to the rigid and inflexible display unit. One of the problems in the prior art solutions has been how to make waterproof display units for current watch-like displays, for example; problems relating to waterproofness are caused, for example, by the joints between the display glass and the face of the watch and those between the clockwork mechanism and the background plate of the watch. A watch according to the preamble of claim 1 is disclosed in FR 2 595 480.

### BRIEF DESCRIPTION OF THE INVENTION

It is an object of the invention to provide an improved wrist-worn device structure. This is achieved with a wrist-worn device of the invention which comprises a wristband to be arranged around the wrist and an electronics unit in connection with the wristband, the electronics unit being arranged to process a value of an exercising variable relating to a physical activity, the wrist-worn device further comprising a display for showing the exercising variable value. The wristband, electronics unit and display of the wrist-worn device are integrated to form a single, uniform wrist-worn device.

The preferred embodiments of the invention are disclosed in the dependent claims.

The invention thus relates to a novel wrist-worn device solution. An underlying idea of the invention is that the wristband, the display and the electronics unit are integrated to form a single entity, i.e. a uniform piece. Integration as used in the present specification means that instead of being united after the manufacturing phase, the separate parts of the device are joined together process-technically during the manufacturing phase, for example by injection moulding, in which the parts are coated with plastic to produce a uniform piece. The parts thus form a wrist-worn device in which the display and the electronics unit can be thought of as integral parts of the wristband with which the device can be attached to the wrist. With injection moulding, the wrist-worn device can be manufactured in a plural number of phases, whereby different plastics with different transparency and colour properties can be used. For example, the part of the wrist-worn device that encapsulates the display is made using transparent plastic.

In the context of this specification, the term wrist-worn device refers to heart rate monitors, wrist-worn computers, watches, and similar devices worn on the wrist. A heart rate monitor is a device comprising a wrist-worn device the functions of which include at least reception of heart rate information and display of the heart rate formed on the basis of the information. A wrist-worn computer, in turn, is a device the functions of which include at least user positioning. In the present invention, the display of the wrist-worn device shows at least one exercise parameter representing a physical activity. The exercise parameter to be displayed is preferably the heart rate or a heart rate variable derived from the heart rate, such as heart rate variation, maximum rate, minimum rate. The exercise variable may also be for example time measured from the beginning of the exercise, remaining exercise time or time measured within a specific range of heart rate. It is also possible to display multiple exercise variables to the user, for example heart rate and time from the beginning of the exercise. According to a preferred embodiment of the invention, the wrist-worn device comprises a number of displays equal to the number of exercise variables to be displayed. The device carries out exercise variable processing, which in the case of heart rate, for example, means that heart rate information is received from a heart rate transmitter / the electrodes of the wrist-worn device and the heart rate is calculated on the basis of the received heart rate information. The processing also covers the transmission of the heart rate information received from the heart rate transmitter for display.

The electronics part of the wrist-worn device preferably comprises means for receiving heart rate information and means for forming a person's heart rate on the basis of the heart rate information. The heart rate information is preferably received as inductive transmission from the electrode belt or as a measurement carried out by sensors of the wrist-worn device.

In this context, the term display refers to the parts of the device used for visually displaying an exercise variable value to the user. In a preferred embodiment of the invention, the display is composed of display areas. The display is preferably formed on a resilient and flexible circuit board, and each display area corresponding to a specific exercise variable value can be illuminated with a display-area-specific illumination element, such as a LED (Light Emitting Diode). The invention is not restricted to an arrangement where the illumination elements would be close together, but they may be arranged on various locations on the wristband. Consequently, the display is not necessarily a single, uniform rigid display unit such as those of the known solutions, but it may consist of a plural number of display parts. The number of display areas used for displaying the exercise variable values is naturally not limited in the invention either. The display areas may take various shapes; for example, they may be in the form of a rectangle, circle or ellipsis. The topmost layer of the display is made of a translucent plastic to make the light emitted by a display-area-specific light source visible to the user. The display is preferably a bar display comprising a string of display areas arranged along a substantially straight line. A first end of the bar display comprises a value range corresponding to the lowest exercise variable values that can be displayed, a second end comprising in turn a value range corresponding to the highest exercise variable values that can be displayed. An exercise variable value can be displayed for example by only illuminating the display area to which the exercise variable value relates. Alternatively, all value ranges lower than said value range are illuminated, in addition to the value range concerned. Instead of a bar display, a display forming an arc, for example, can also be used.

An advantage of the invention is that it provides an improved wrist-worn device solution in which the material encapsulating the device components enhances the waterproofness of the device. A wrist-worn device made as a uniform, single-piece construction provides manufacture-technical advantages both in view of costs and durability.

### BRIEF DESCRIPTION OF THE DRAWINGS

In the following, the invention will be described with reference to the accompanying drawings, in which
Figure 1 shows a top view of an embodiment of a wrist-worn device;
Figure 2 shows a sectional side view of a preferred embodiment of the wrist-worn device;
Figure 3 shows a display according to an embodiment of the invention;
Figure 4 illustrates some embodiments of a heart rate transmitter and the wrist-worn device.

### DESCRIPTION OF EMBODIMENTS

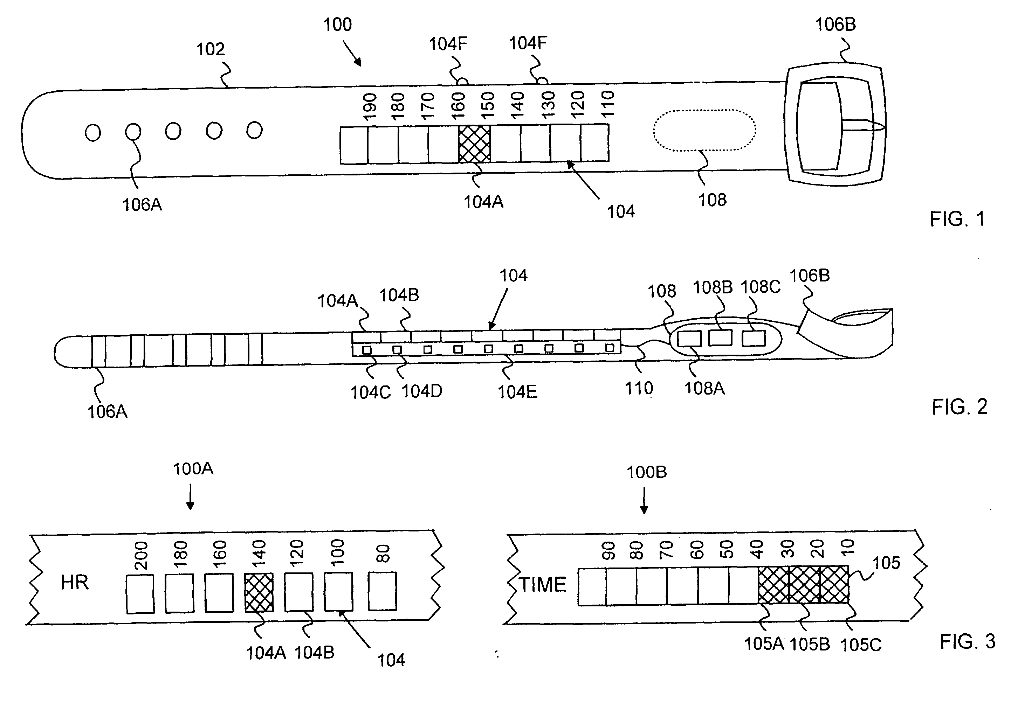

In the following, the invention will be described with reference to preferred embodiments and the accompanying Figures 1-4. Figure 1 shows a wrist-worn device 100 according to a preferred embodiment of the invention. The wrist-worn device 100 comprises a wristband 102 and a display 104 showing a momentary value of an exercise variable. In the example of Figure 1, the momentary exercise variable value, i.e. heart rate, is shown on a display area 104A. In the Figure, the display area 104A is illuminated to show the user that his heart rate is between 150 and 160. The invention is naturally not limited to the number of the display areas, i.e. whether there is a display area for each value range of 2 beats, 5 beats or 10 beats. The colours of the display areas are not restricted in the invention either. For example, display areas of heart rate levels below 100 may be yellow, at levels between 100 and 150 they may be green and at levels above 150 they may be red. The different colours are produced using LEDs of different colours, for example, or by coating the display areas concerned with plastics of different colours. The wrist-worn device further comprises attaching means, i.e. holes 106A and 106B, for attaching the device to the wrist. Instead of a buckle attachment, the wrist-worn device may be attached to the wrist using Velcro, for example, or some other known means. The functions of the wrist-worn device 100 are controlled with input keys 104F which are implemented for example as push-buttons, turn-buttons or membrane keys. The wrist-worn device 100 further comprises an electronics unit 108 which is integrated into the construction and therefore shown with a broken line in Figure 1.

Figure 2 shows a sectional side view of the wrist-worn device 100 of Figure 1. The display 104 is divided into display areas of which the Figure shows two: display areas 104A and 104B. The display area 104A is illuminated with a light source 104C and the display area 104B with a light source 104D. The light sources 104C-104D are arranged onto a flexible circuit board 104F which forms the base of the display. Because of the circuit board 104E, the display 104 as a whole is flexible and bends at least partly around the user's wrist when the device is worn on the wrist. The advantage of this is that the display is not limited solely to a rigid display unit visible on the top side of the wrist, i.e. the side facing away from the palm side of the hand. When built on a flexible circuit board, the display of the wrist-worn device can be extended so as to be visible also on the sides of the wrist and, at least partly, even on the palm side of the hand. The electronics unit 108 comprises a power source 108A, such as a battery, for producing the current needed by the electronics unit 108 and the display 104. At the bottom of the wrist-worn device 100, for example, there is provided an opening for the battery 108A through which opening the battery can be changed when necessary. The electronics unit further comprises means for receiving heart rate information 108B and means for forming a heart rate 108C on the basis of the heart rate information for display on a display 104B of the wrist-worn device. For the transfer of electric current and information, the display 104 of the device 100 and the electronics unit are interconnected via a connecting line 110 or the flexible circuit board 104E, for example. The user interface is a menu-type hierarchical system, for example, in which push-buttons 104F are used for making selections and for activating and stopping functions, such as a heart rate measurement.

Figure 2 shows the structure of the wrist-worn device 100 in which the display 104, wire 110 and electronics unit 108 are encapsulated in the wristband 102. The display 104 and the electronics unit 108 are sealed into the wristband 102, i.e. there are no open seams in the solution of the invention between the wristband and said components. This enhances the waterproofness of the device because components sensitive to water are entirely surrounded by plastic. The components are encapsulated to form a uniform piece by injection moulding, for example, which is a technique in which molten plastic mass is injected into a mold and cooled to produce a durable, flexible, sealed and uniform wrist-worn device. At least at the display 104 the plastic cover encapsulating the wrist-worn device 100 is made of transparent plastic to allow the display 104 to be made visible. An injection moulding process in which different plastics and colours are used is technically possible by encapsulating the wrist-worn device in several stages and changing the plastic between the stages. Although in Figure 2 the top surface / exterior face of the display 104 is level with the top surface of the device 100, the solution of the invention is not restricted to this, but the wrist-worn device 100 may also be implemented such that half of the display 104 is inside the area defined by the wristband, the display 104 thus partly rising above the level of the wristband 102. It is also possible to position light sources into the device such that they embedded into it, extending only to the level of the surface of the device. The light sources are thus otherwise encapsulated in plastic except for the part visible to the user.

Figure 3 illustrates, by way of example, two alternatives for showing exercise information on the display of the wrist-worn device. In the example, two exercise variable values are displayed to the user, the user's heart rate on a wrist-worn device 100A and the duration of the exercise on a wrist-worn device 100B. The above exercise variables could naturally also be displayed using one wrist-worn device comprising two displays, one for each exercise variable. In the device 100A only one display area 104A is illuminated to show that the user's heart rate is within 140-160. The display areas 104A and 104B of the display of the device 100A are not arranged to be in immediate contact with each other, but separated by the plastic encapsulating the device 100A. The display areas 104A and 104B are thus shown as separate luminous points on the device. In the wrist-worn device 100B, the display areas 105A-105C are close together and a momentary value is indicated by illuminating the display area 105A representing the value and the display areas 105B-105C indicating values lower than the momentary value. The displays shown in Figures 1-3 may also be other than bar-shaped; for example, they may form a full circle or a half-circle.

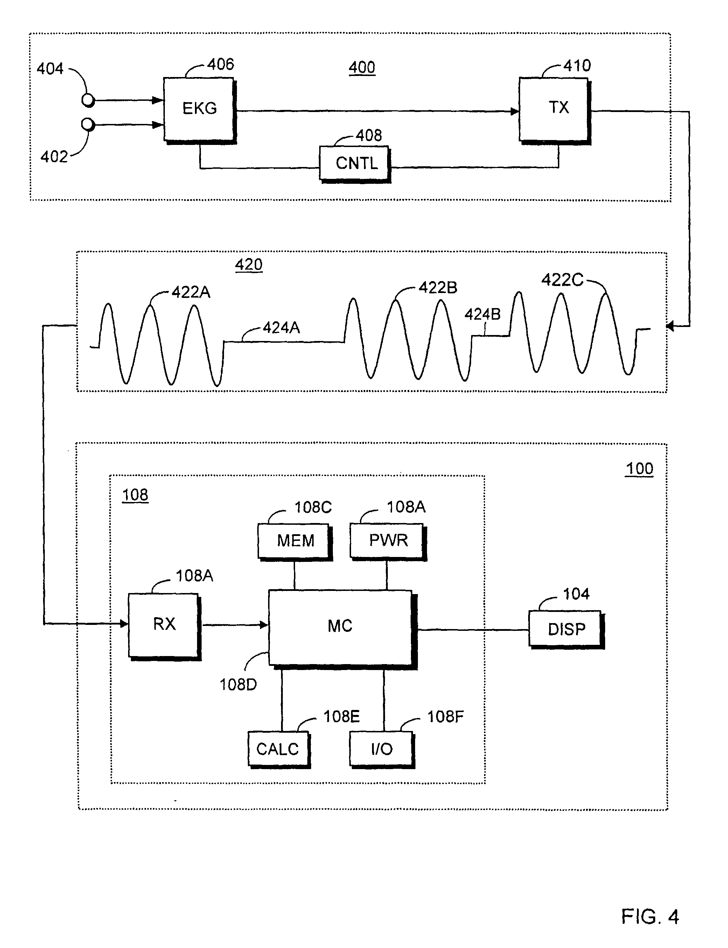

Figure 4 is a block diagram illustrating the structure of a heart rate transmitter-receiver pair. The Figure only shows the essential parts of the heart rate transmitter, such as an electrode belt 400 to be placed on the chest, and a wrist-worn receiver 100, although a person skilled in the art will find it apparent that other parts may also be included but it is not relevant to describe them in this context. The electrode belt 400 comprises an electronics unit 408 which receives the heart rate information from measurement electrodes 402, 404, which in turn produce an EKG signal measurement by measuring the difference in potential between the electrodes 402-404. The EKG signals are preferably processed, i.e. filtered, amplified and detected, in an EKG detection block 406 using prior art methods to allow heartbeats to be detected from the signal. The detection of heartbeats is based on a QRS complex detected in the heartbeat signal, for example, the letters Q, R and S referring to potential phases caused in an electric signal by an electric activation of the heart. The QRS may be detected in the EKG detection block using a matched filter, for example, whereby a model complex is compared with a measured QRS complex and when the comparison exceeds a predetermined threshold value, the complex is accepted as a heartbeat. Heart rate information 420 is transmitted from the electrode belt 400 to the wrist-worn device 100 using the transmitter 410, which is implemented as a coil, for example.

In the heart rate information 420 to be transmitted, one heartbeat or heart rate data bit is represented by one 5 kHz burst 422A, for example, or a group 422A, 422B, 422C of several bursts. Intervals 424A, 424B between the bursts may be of an equal duration, or their duration may vary. The heart rate information 420 to be transmitted may consist of heartbeat information, as described above, or the heartbeats may be processed already in the transmitter to form computational exercise variables, such as an average heart rate or heart rate distribution. The computational variables can naturally also be formed in the wrist-worn device, on the basis of the heart rate information. The information 420 may be transmitted inductively, or it may be sent optically or through a wire. The wrist-worn device 100 and its electronics unit 108 in particular comprise receiver means 108B, such as a coil. A signal received with the receiver means 108B is supplied to control electronics 108D which control and coordinate the operation of the electronic parts of the wrist-worn device 100. The control electronics 108C together with the related memory are preferably implemented using a general-purpose microprocessor provided with the necessary system and application software, although diverse hardware implementations are also possible, such as a circuit built of separate logic components, or one or more ASICs (Application Specific Integrated Circuit).

The wrist-worn device 100 comprises electric current produced for the electronics unit 108 and the display by a power source 108A. The device 100 preferably comprises a memory 108C for storing the received heart rate information 420 and the computer software of the device 100. The received heart rate information 420 is processed in a computation unit 108E of the electronics unit 108 to produce the user's heart rate and/or other heart rate variables for display on the display 104 connected to the electronics unit 108. The wrist-worn device 100 preferably comprises a user interface 108F for supplying information to the device 100 and for transferring information stored on the device 100 to an external computer, for example, for further processing. Input functions at the user interface 108F are implemented for example using push-buttons and/or turn-buttons for making selections and for activating and stopping functions. The input functions can also be implemented using membrane keys, in which case the display of the wrist-worn device is pressed with the finger, selecting pen or a similar means to make selections. In addition, the user interface 108F preferably comprises means for producing sound signals to provide alarms informing when an exercise variable is exceeded. The user interface 108F, such as a telecommunications port, can also be used for updating the software of the wrist-worn device.

Although the heart rate monitor described with reference to Figure 4 comprises an electrode belt to be placed on the chest and a device to be carried on the wrist, a heart rate monitor composed of a single-piece wrist-worn device is also possible. In that case the device comprises pressure sensors to measure heart rate information from the blood circulation in a vein, or optical sensor for an optical heart rate measurement from the circulation of blood in a vein. When a single-piece device is concerned, heart rate information is transmitted from the sensors to the electronics unit using conductive plastic or a connecting wire, for example.

Although the invention is described above with reference to examples according to the accompanying drawings, it is apparent that the invention is not restricted to them, but may vary in many ways within the scope disclosed in the claims.

## Claims

1. A wrist-worn device (100) comprising a wristband (102) to be arranged around the wrist and an electronics unit (108) in connection with the wristband (102), the wrist-worn device (100) further comprising a display (104), the wristband (102), electronics unit (108) and display (104) of the wrist-worn device (100) being encapsulated together to form a single, integral wrist-worn device (100) **characterized in that** the electronics unit is arranged to process a value of an exercising variable relating to a physical activity, the display (104) being arranged to show the exercising variable value, and the display is a bar display comprising a string of display areas and is constructed onto a flexible circuit board to make the display and the wrist-worn device flexible.

2. A wrist-worn device according to claim 1, **characterized in that** the wristband, display and electronics unit of the device are sealed and integrated together with plastic to form a uniform piece.

3. A wrist-worn device according to claim 2, **characterized in that** the wristband, display and electronics unit of the device are integrated together with plastic by injection moulding.

4. A wrist-worn device according to claim 2, **characterized in that** the portion of the device encapsulating the display is formed of transparent plastic.

5. A wrist-worn device according to claim 1, **characterized in that** one or more of the following serve as exercise variables: the person's heart rate, average heart rate, heart rate deviation, exercise duration from the beginning of the exercise.

6. A wrist-worn device according to claim 1, **characterized in that** the electronics unit comprises receiver means for receiving heart rate information and computing means for processing the heart rate information to show the person's heart rate on the display.

7. A wrist-worn device according to claim 1, **characterized in that** each display area of the display is used for showing a value range containing at least one possible exercise variable value.

8. A wrist-worn device according to claim 1, **characterized in that** the display area at a first end of the bar display is for the value range representing the lowest exercise variable value to be displayed and the display area at a second end is for the value range showing the highest exercise variable value to be displayed.

9. A wrist-worn device according to claim 1, **characterized in that** the display comprises an illumination element for at least one display area to illuminate the display area, the illumination element being arranged to illuminate the display area when a momentary exercise variable value is within the value range corresponding to the display area in question.

10. A wrist-worn device according to claim 1, **characterized in that** the value ranges on the bar display are arranged on a substantially straight line with respect to one another.

## Patentansprüche

1. Am Handgelenk getragene Vorrichtung (100) mit einem Armband (102) für die Anordnung um das Handgelenk und mit einer Elektronikeinheit (108) in Verbindung mit dem Armband (102), wobei die am Handgelenk tragbare Vorrichtung (100) ferner eine Anzeige (104) aufweist und das Armband (102), die Elektronikeinheit (108) und die Anzeige (104) der am Handgelenk tragbaren Vorrichtung (100) zur Bildung einer einzigen integralen am Handgelenk tragbaren Vorrichtung (100) zusammen eingekapselt sind, **dadurch gekennzeichnet, dass** die Elektronikeinheit angeordnet ist, um einen Wert einer Trainingsvariablen bezogen auf eine körperliche Aktivität zu verarbeiten, die Anzeige (104) angeordnet ist, um den Wert der Trainingsvariablen zu zeigen, und die Anzeige eine Stabanzeige mit einem Strang von Anzeigebereichen und auf einer flexiblen Leiterplatte aufgebaut ist, um die Anzeige und die am Handgelenk tragbare Vorrichtung flexibel zu machen.

2. Am Handgelenk tragbare Vorrichtung nach Anspruch 1, **dadurch gekennzeichnet, dass** das Armband, die Anzeige und die Elektronikeinheit der Vorrichtung zur Bildung eines Einheitsteils mit Kunststoff abgedichtet und zusammengefügt sind.

3. Am Handgelenk tragbare Vorrichtung nach Anspruch 2, **dadurch gekennzeichnet, dass** das Armband, die Anzeige und die Elektronikeinheit der Vorrichtung durch Spritzgießen mit Kunststoff zusammengefügt sind.

4. Am Handgelenk tragbare Vorrichtung nach Anspruch 2, **dadurch gekennzeichnet, dass** der Teil der Vorrichtung, in dem die Anzeige eingekapselt ist, von einem transparenten Kunststoff gebildet wird.

5. Am Handgelenk tragbare Vorrichtung nach Anspruch 1, **dadurch gekennzeichnet, dass** eine oder mehrere der folgenden Variablen als Trainingsvariable einer Person dienen: die Herzfrequenz, die mittlere Herzfrequenz, die Herzfrequenzabweichung, die Trainingsdauer vom Beginn des Trainings.

6. Am Handgelenk tragbare Vorrichtung nach Anspruch 1, **dadurch gekennzeichnet, dass** die Elektronikeinheit Empfängereinrichtungen zum Empfangen der Herzfrequenzinformation und Recheneinrichtungen zum Verarbeiten der Herzfrequenzinformation aufweist, um an der Anzeige die Herzfrequenz der Person zu zeigen.

7. Am Handgelenk tragbare Vorrichtung nach Anspruch 1, **dadurch gekennzeichnet, dass** jeder Anzeigebereich der Anzeige dazu verwendet wird, einen Wertebereich zu zeigen, der wenigstens einen Wert einer möglichen Trainingsvariablen enthält.

8. Am Handgelenk tragbare Vorrichtung nach Anspruch 1, **dadurch gekennzeichnet, dass** der Anzeigebereich an einem ersten Ende der Stabanzeige für den Wertebereich dient, der den anzuzeigenden Niedrigstwert der Trainingsvariablen darstellt, und dass der Anzeigebereich an einem zweiten Ende für den Wertebereich dient, der den anzuzeigenden Höchstwert der Trainingsvariablen zeigt.

9. Am Handgelenk tragbare Vorrichtung nach Anspruch 1, **dadurch gekennzeichnet, dass** die Anzeige ein Beleuchtungselement für wenigstens einen Anzeigebereich aufweist, um den Anzeigebereich zu beleuchten, wobei das Beleuchtungselement so angeordnet ist, dass es den Anzeigebereich beleuchtet, wenn sich ein MomentanWert für die Trainingsvariable innerhalb des Wertebereichs befindet, der dem fraglichen Anzeigebereich entspricht.

10. Am Handgelenk tragbare Vorrichtung nach Anspruch 1, **dadurch gekennzeichnet, dass** die Wertebereiche an der Stabanzeige auf einer im Wesentlichen geraden Linie bezüglich einander angeordnet sind.

## Revendications

1. Dispositif porté au poignet (100) comprenant un bracelet (102) devant être agencé autour du poignet et une unité électronique (108) reliée au bracelet (102), le dispositif porté au poignet (100) comprenant en outre un afficheur (104), le bracelet (102), l'unité électronique (108) et l'afficheur (104) du dispositif porté au poignet (100) étant contenus ensemble afin de former un seul dispositif porté au poignet intégral (100), **caractérisé en ce que** l'unité électronique est agencée pour traiter une valeur d'une variable d'exercice relative à une activité physique, l'afficheur (104) étant agencé pour indiquer la valeur de la variable d'exercice, et l'afficheur est un afficheur à barres comprenant une ligne de zones d'affichage et est construit sur une carte de circuit. imprimé flexible afin que l'afficheur et le dispositif porté au poignet soient flexibles.

2. Dispositif porté au poignet selon la revendication 1, **caractérisé en ce que** le bracelet, l'afficheur et l'unité électronique du dispositif sont scellés et intégrés à du plastique afin de former une pièce uniforme.

3. Dispositif porté au poignet selon la revendication 2, **caractérisé en ce que** le bracelet, l'afficheur et l'unité électronique du dispositif sont intégrés à du plastique par moulage par injection.

4. Dispositif porté au poignet selon la revendication 2, **caractérisé en ce que** la partie du dispositif contenant l'afficheur est formée en plastique transparent.

5. Dispositif porté au poignet selon la revendication 1, **caractérisé en ce qu'**un ou plusieurs des éléments suivants sert/servent de variables d'exercice : le rythme cardiaque de la personne, le rythme cardiaque moyen, l'écart de rythme cardiaque, la durée de l'exercice depuis le début de l'exercice.

6. Dispositif porté au poignet selon la revendication 1, **caractérisé en ce que** l'unité électronique comprend un moyen de réception permettant de recevoir des informations relatives au rythme cardiaque, et un moyen de calcul permettant de traiter les informations relatives au rythme cardiaque afin d'indiquer le rythme cardiaque de la personne sur l'afficheur.

7. Dispositif porté au poignet selon la revendication 1, **caractérisé en ce que** chaque zone d'affichage de l'afficheur est utilisée pour indiquer une gamme de valeurs contenant au moins une valeur de variable d'exercice possible.

8. Dispositif porté au poignet selon la revendication 1, **caractérisé en ce que** la zone d'affichage située à une première extrémité de l'afficheur à barres est destinée à la gamme de valeurs représentant la valeur de variable d'exercice la plus faible pouvant être affichée, et la zone d'affichage située à une seconde extrémité est destinée à la gamme de valeurs indiquant la valeur de variable d'exercice la plus élevée pouvant être affichée.

9. Dispositif porté au poignet selon la revendication 1, **caractérisé en ce que** l'afficheur comprend un élément d'éclairage destiné à au moins une zone d'affichage afin d'éclairer la zone d'affichage, l'élément d'éclairage étant agencé pour éclairer la zone d'affichage lorsqu'une valeur de variable d'exercice momentanée se trouve dans la gamme de valeurs correspondant à la zone d'affichage en question.

10. Dispositif porté au poignet selon la revendication 1, **caractérisé en ce que** les gammes de valeurs de l'afficheur à barres sont agencées sur une ligne sensiblement droite les unes par rapport aux autres.
